# EUROPEAN PATENT SPECIFICATION

(11) **EP 1 159 257 B1**
(45) Date of publication and mention of the grant of the patent: **21.04.2004**
(21) Application number: 00909277.6
(22) Date of filing: 28.02.2000
(51) Int. Cl.: C07C 219/06, C07C 219/08, C07C 215/24, C11D 1/62

(54) **ESTERQUATS, THEIR INTERMEDIATES, A PROCESS TO MAKE THE ESTERQUATS, AND THEIR USE**
ESTERQUATS, DEREN ZWISCHENPRODUKTEN, VERFAHREN ZU IHRER HERSTELLUNG UND IHRE VERWENDUNG
COMPOSES QUATERNAIRES D'ESTER ET LEURS INTERMEDIAIRES; PROCEDE DE FABRICATION DE CES COMPOSES ET LEUR UTILISATION

(30) Priority: 05.03.1999 EP 99200638
(43) Date of publication of application: 05.12.2001
(73) Proprietor: Akzo Nobel N.V., 6824 BM Arnhem (NL)
(72) Inventor: AHRENS, Hartmut, D-60598 Frankfurt (DE); BERGFELD, Manfred, Josef, D-63906 Erlenbach-Mechenhard (DE)
(74) Representative: Schalkwijk, Pieter Cornelis
(86) International application number: PCT/EP2000/001738
(87) International publication number: WO 2000/053570

(56) References cited:
- EP-A- 0 330 261
- WO-A-97/13743
- WO-A-97/47588

## Description

The invention relates to specific quaternary ammonium compounds having at least one nitrogen-bonded moiety with at least one ester function (esterquats), to intermediates for making such esterquats, to compositions comprising one or more of these esterquats, to a process to make them, and to the use of the esterquats as a fabric softener.

Quaternary ammonium compounds having a substituent on the nitrogen atom with two ester groups are known. Also compositions comprising such ammonium compounds are known. WO 97/47588, for instance, discloses how 2,3-dihydroxypropyl trimethyl ammonium chloride is reacted with lauric acid to form a composition comprising the corresponding diester quaternary compound, a propyl-diester-quat (PDQ).

The use of PDQ as a fabric softener is known to result in a very good softening performance. However, the manufacture of these compounds is cumbersome and the raw material N,N-dimethyl-1-amino propane-2,3-diol is costly. Also, the epichlorohydrin typically used to make this raw material for PDQ is less desired from an environmental viewpoint. Furthermore, it is noted that the production of PDQ typically requires the use of a solvent, such as isopropanol, in the quaternization reaction. However, such solvent can transesterify with the PDQ, resulting in the formation of a contaminant (the fatty acid derivative of the solvent) and a reduction of the softening performance of the PDQ-containing product (because less diester compound is present). Therefore, there is a need for alternative compounds with a better price/performance that can be produced according to a process that puts less strain on the environment. Preferably, this process does not require the use of solvents that can transesterify with fatty acid esters.

Our investigations have led us to a new and surprisingly simple process for making new types of quaternary compounds where at least one of the nitrogen substituents contains at least one ester group, and to new intermediates. These compounds, and compositions comprising these compounds, offer a good fabric softening performance and a better price/performance than the conventional fabric softeners. It is noted that the intermediates may be used as fabric softeners themselves. Also, the new process offers advantages from an environmental viewpoint because epichlorohydrin is not used. Furthermore, in one of the preferred embodiments the quaternization step of the amine is not performed towards the end of the synthesis of the surface-active compound, but already on the low-molecular weight amine. The process is improved by this, because it is much simpler to separate and purify the low-molecular weight quaternized amine than to perform such steps on the resulting surface-active compound, if so desired. More specifically, during the processing/washing of a surface-active compound, there is a tendency on the part of the compound to disperse. In another preferred embodiment, a trialkylamine is reacted with an epoxy alkene in the presence of an acid, in order to produce a quaternary intermediate. The process of this preferred embodiment obviates the use of epichlorohydrin as well as the use of undesired solvents. The quaternary intermediate can be esterified to give the preferred diesterquat fabric softeners. However, the monoesterquat that is formed may be useful as a fabric softener as well.

The new quaternary compounds according to the invention are of the formula:

R₄[R₅R₆N⁺Z]ₙ X⁻,

wherein Z is covalently bonded to the nitrogen atom, and of the following formulae (I-IV) 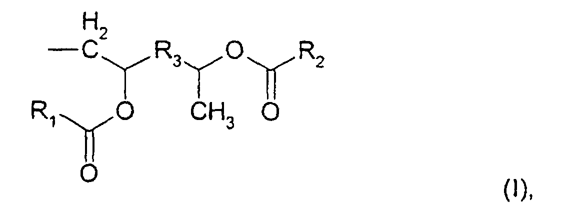 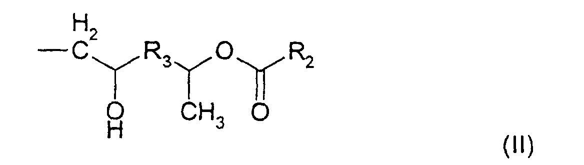 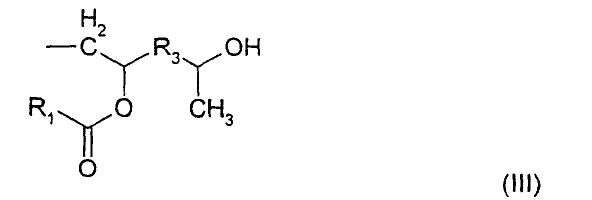 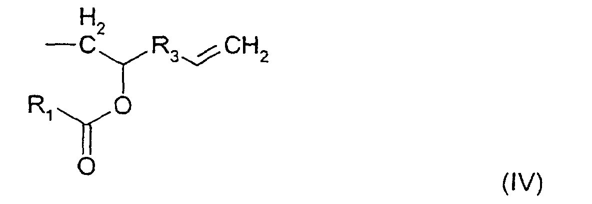 or isomers thereof with the formulae: 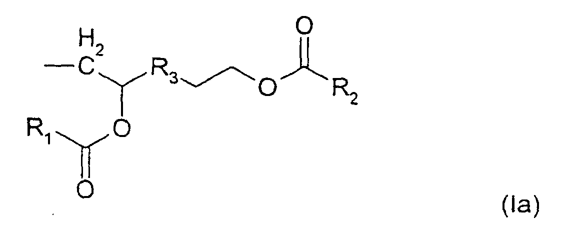 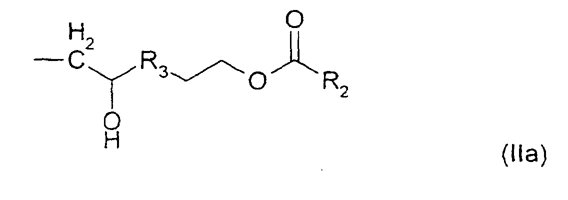 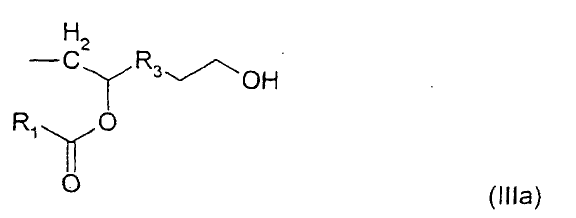 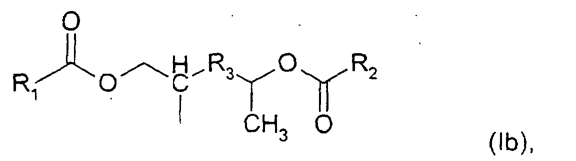 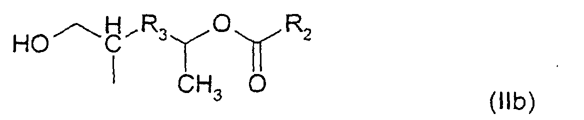 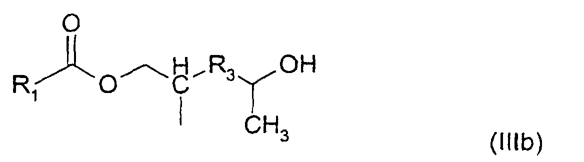 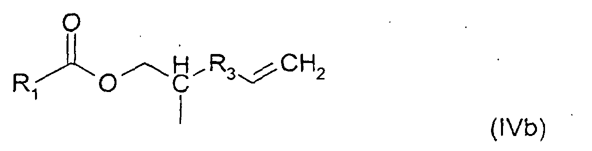 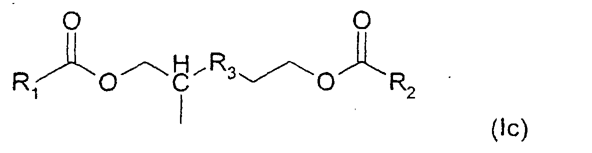 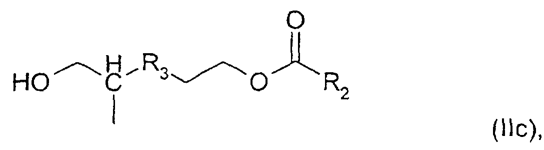 and 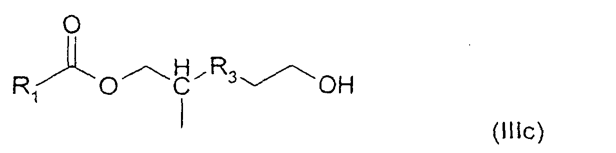 wherein,
R₁ and R₂ are independently selected from linear or branched, saturated or unsaturated C₆₋₂₂ hydrocarbyl,
R₃ is nothing or C₁₋₂₀ hydrocarbyl,
R₄ is C₁₋₆ alkyl, C₁₋₆ alkylene, or independent Z,
R₅ is H, C₁₋₆ alkyl, independent Z, or the residue of the quaternizing agent, such as C₁₋₃₀ alkyl or alkenyl, preferably, C₁₋₇ alkyl or alkenyl,
R₆ is C₁₋₆ alkyl or independent Z,
n is 1, 2 or 3 and
X⁻ is an ion selected from Cl⁻, Br⁻, I⁻, F⁻, CH₃SO₄⁻, C₂H₅SO₄⁻, H₂PO₄⁻, HPO₄²⁻, PO₄³⁻, H₂PO₃⁻, HPO₃²⁻, H₂PO₂⁻, HPO₂²⁻, nitrate⁻, formate⁻, acetate⁻, propionate⁻, tartrate⁻ and benzoate⁻, wherein the total charge of the anions equals the total charge of the cations. The compounds may be used after purification and/or isolation. Preferably, they are part of a composition comprising more than one of the compounds of formulae I-IV and isomers thereof, since extensive isolation steps for the individual compounds can then be refrained from. Purification may include a bleaching and/or adsorption step to convert and/or remove chemicals that cause discolouration.

Preferred are compounds of formula (I), given in full below 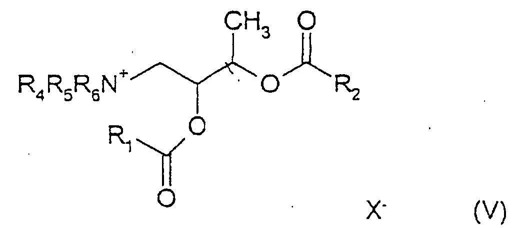 or isomers thereof, wherein R₁-R₆ have the meaning as presented above.

More preferably, the compounds of the above formulae I-IV, or the isomers thereof, are of the formula wherein R₁ and R₂ independently selected from linear or branched, saturated or unsaturated are C₁₂₋₁₈ alkyl. Even more preferably, R₁ and R₂ have a carbon distribution within the range such as can be found in commercial fatty acids. Also preferred compounds of formulae I-IV or isomers thereof are those of the formula wherein R₄ and R₆ are methyl or ethyl.
Other preferred compounds of formulae I-IV or isomers thereof are those of the formula wherein R₅ is methyl or ethyl.
Preferably, n is 1.
Further preferred compounds of formulae I-IV or isomers thereof are those of the formula wherein X⁻ is chloride, methyl sulfate, or ethyl sulfate.

Another embodiment of the invention is the process to make the compounds according to formulae I-IV and/or isomers thereof. This process involves the reaction of an unsaturated epoxide with an amine, preferably a dialkylamine or trialkylamine, after which the unsaturated, hydroxy group-substituted intermediate is reacted with, on average, 1-2 moles of fatty acid groups per mole of hydroxy group of the intermediate to form an ester. Using this ratio of fatty acid groups will ensure that at least some of the unsaturated bonds are reacted as well to form the preferred diesters.

It is noted that F.F. Blicke and J.H. Biel in *J. Am*.*Chem*.*Soc*. 79, 5508-5512 (1957) disclose that 1,2-epoxy-3-butene can be reacted with aqueous dimethylamine hydrochloride to form 1-dimethylamino-2-hydroxy-3-butene. However, it is not disclosed that such a compound can be further reacted with 1-2 moles of fatty acid per mole of this product to form the compounds according to the invention with good fabric softening performance.

Another embodiment of the invention concerns amines that can be formed as intermediates by reacting a dialkylamine with an unsaturated epoxide, followed by esterification. These intermediates are of the formula R₄[R₆NZ]ₙ, wherein R₄, R₆, n, and Z have the meaning specified above. These intermediates may be used as fabric softeners themselves. However, preferably they are quaternized in conventional ways with agents of the formula R₅X, in order to give the preferred products of the formulae I-IV and isomers thereof.

Intermediate products formed by reacting a trialkylamine with an unsaturated epoxide in the presence of an acid are preferably of the formula 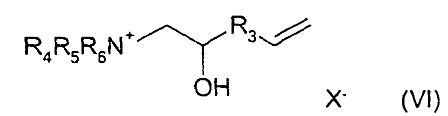 or the isomers thereof according to the formula 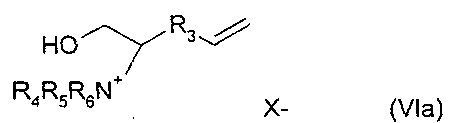 wherein R₃-R₆ and X-have the meaning specified above.
These intermediates can be easily transformed into the desired compounds of formulae I-IV by direct or trans-esterification. The products of formula VI, or the isomers thereof, may themselves be used as a fabric softener. Therefore, a composition comprising compounds I-IV may also contain intermediates of formula VI, without the fabric softening properties being adversely affected.

A further embodiment of the present invention constitutes the use of the compounds according to formulae I-VI, or the isomers thereof, as a fabric softener. Preferably, the compounds are compounds of formula I or the isomers thereof. The products are well-suited for this use because of their expected biodegradability, due to the two ester functions in the molecule, and their price/performance. It is noted that it is known in the art to further improve the performance of fabric softening compositions by combining the fabric softening compounds with a performance booster selected from the group consisting of cationic and non-ionic surfactants. When used in this fashion, the fabric softening compounds are even more effectively deposited on the textile fabric.

The amine that is reacted with the unsaturated epoxide is of the structure R₄[R₅R₆N]ₙ, or the protonated form thereof with structure R₄[R₅R₆N⁺H]ₙ X⁻, wherein R₄, R₅, R₆, n, and X⁻ have the meaning defined above. Preferably, a dialkylamine or trialkylamine is reacted with the unsaturated epoxide. Preferably, n is 1. Of the amines with n is 2, ethylene diamine is preferred.

The unsaturated epoxides that can be used are preferably 1,2-epoxyalkenes of the formula 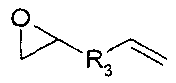 wherein R₃ has the meaning defined above.
Preferred 1,2-epoxyalkenes are 1,2-epoxy-3-butene, 1,2-epoxy-4-pentene, 1,2-epoxy-5-hexene, 1,2-epoxy-6-heptene, 1,2-epoxy-3-pentene, 1,2-epoxy-3-hextene, 1,2-epoxy-3-heptene, and 1,2-epoxy-4-hexene. More preferred 1,2-epoxyalkenes are 1,2-epoxy-3-butene, 1,2-epoxy-3-pentene, 1,2-epoxy-3-hexene, and 1,2-epoxy-3-heptene. Most preferred is 1,2-epoxy-3-butene.

At least n mole of 1,2-epoxyalkene is to react with one mole of R₄[R₅R₆N]ₙ, or the protonated form thereof with structure R₄[R₅R₆N⁺H]ₙ X⁻, according to the invention. However, if a monoalkylamine or ammonia is used to make the indicated dialkylamine or trialkylamine *in situ* by first reacting it with 1,2-epoxyalkene, then up to four moles of 1,2-epoxyalkene can be reacted per mole of amine.

The ester functions in the compounds of the invention typically are derived from saturated or unsaturated, linear or branched C₆₋₃₀, preferably C₈₋₂₂, more preferably C₁₂₋₁₈, most preferably naturally occurring, fatty acids, optionally substituted with, e.g., one or more hydroxy groups. Preferred fatty acids used in making the compounds of the invention include coconut, palm, palm kernel, soybean, oleic, tallow, rapeseed, canola, behenic, eruca fatty acids, and mixtures thereof. Preferably, a fatty acid mixture comprising at least 50 percent by weight (%w/w) of C₁₂₋₁₈ fatty acids is used.
The acids may be used as such in a conventional direct esterification process, but also derivatives can be used, such as the corresponding acid chlorides or (mixed) anhydrides. In transesterification reactions typically a fatty acid ester is used. In such transesterification reactions preferably the methyl, ethyl and/or glycerol esters of the acids are used. Most preferred are mono-, di- and/or triglycerides of the acids. In the esterification process preferably a conventional (trans)esterification catalyst is used, such as hypophosphorous acid.

Preferably 50-100 percent, on a molar basis, of the hydroxy functions of the 1-amino-2-hydroxy-alkene intermediate formed after reacting amine and 1,2-epoxyalkene is esterified. More preferably, 75-100 mole percent of the hydroxy groups is esterified. Also, preferably 50-100 percent, on a molar basis, of the unsaturated functions of the 1-amino-2-hydroxy-alkene intermediate is reacted with fatty acid to give an ester function. More preferably, 75-100 mole percent of the alkene functions is esterified.

If a dialkylamine is used in the reaction with the 1,2-epoxyalkene, then the resulting diester bearing amine is to be quaternized in order to achieve the preferred fabric softening compounds according to the invention. The quaternization step is conventional, using agents of the formula R₅X, wherein R₅ and X have the meaning defined above. Examples of conventional quaternizing agents include, but are not limited to, dimethyl sulphate, diethyl sulphate, methyl chloride, methyl bromide, methyl iodide, benzyl chloride, benzyl bromide, allyl chloride, and allyl bromide.
However, in a preferred embodiment of the invention, the raw material is not a dialkylamine but a trialkylamine. When such trialkylamines are reacted with the unsaturated epoxide in the presence of a conventional activator for the ring opening of epoxides, typically an acid, such as hydrochloric acid, sulfuric acid, hydrochloric salts of amines, the hydrochloric salt of pyridine, and the like, then the corresponding quaternary ammonium group-bearing unsaturated alcohol is formed. These alcohols can be converted to the corresponding mono- and/or diesterquatemary ammonium compounds by appropriate esterification. The esterification can be a direct esterification or a transesterification, both processes being known in the art for conventional esterification processes. Preferably, C₆₋₂₂ fatty acid groups are introduced into said esterification process, since such groups are needed to obtain the desired fabric softening effect.

The resulting quaternary ammonium compounds, having a nitrogen substituent with at least one ester group, can be used as is or after purification and/or isolation. The compounds, preferably the diester-bearing compounds, are pre-eminently suited for use as a biodegradable fabric softener.

The invention is elucidated by the following, non-optimized, examples.

### Materials used:

Hypophosphorous acid ex Aldrich
Stearic acid ex Merck
Dimethylaminobutenol ex Eastman
Butadiene monoxide (1,2-epoxy-3-butene) ex Aldrich

### Procedure

A mixture of N-(2-hydroxybut-3-en-1-yl)-N,N,N-trimethylammonium chloride and N-(1-hydroxybut-3-en-2-yl)-N,N,N-trimethylammonium chloride was produced by charging a 10 ml flask, equipped with a stirrer, with a mixture of 2.55 g (26.7 mmoles) trimethylamine hydrochloride, 0.06 g (0.5 mmole) of 47.7 %w/w of trimethylamine in water, and 2 g water, and cooling the mixture to 10°C. Then 2.09 g (29.9 mmoles) of 1,2-epoxybutene were added dropwise during 1 hour while the temperature was maintained at 10°C. Thereafter, the mixture was heated to 40°C and further reacted for 2 hours. Subsequently, 1 ml of 1 M HCl was added, after which the water was evaporated at atmospheric pressure. The desired mixture remained.

### Example 1

In a one-necked flask of 100 ml, equipped with stirrer and water separator, 1.58 g (10.2 mmoles) of a mixture of N,N-dimethyl-1-aminobut-3-en-2-ol hydrochloride and N,N-dimethyl-2-aminobut-3-en-1-ol hydrochloride with a purity of 97.5 % by weight (%w/w) was combined with 6.07 g (21.4 mmoles) stearic acid. To the mixture, 1 ml of 50 %w/w of hypophosphorous acid in water was added as a catalyst. The mixture was stirred for 4 hours at 160°C at a reduced pressure of 1 mbar.
A mixture of 2,3-di(stearoyloxy)but-1-yl)dimethylamine hydrochloride and 1,3-di(stearoyloxy)but-2-yl)dimethylamine hydrochloride was formed with a yield of 24 mole%, based on the amino compound.
The product can be quatemized in conventional ways.

### Example 2

In a one-necked flask of 100ml, equipped with stirrer and water separator, 1 ml of 50% by weight hypophosphorous acid in water was added to a mixture of 1.71 g (10.3 mmoles) N-(2-hydroxybut-3-en-1-yl)-N,N,N-trimethylammonium chloride/N-(1-hydroxybut-3-en-2-yl)-N,N,N-trimethylammonium chloride and 5.96 g (21.0 mmoles) stearic acid. With stirring, the mixture was reacted at 160°C for 4 hours under vacuum at a pressure of 1.33 mbar.
A mixture of 2,3-di(stearoyloxy)but-1-yl)trimethyl ammonium chloride (or 1-N,N,N-trimethylammoniumchloride-butane-2,3-distearate) and 1-3-di-(stearoyloxy)but-2-yl)trimethylammonium chloride was formed with a yield of 49 mole%, based on the ammonium compound.
The product has good fabric softening properties.

## Claims

1. Quaternary ammonium compounds of the formula R₄[R₅R₆N⁺Z]ₙ X⁻, wherein Z is covalently bonded to the nitrogen atom and selected from the group of the following formulae (I-IV) 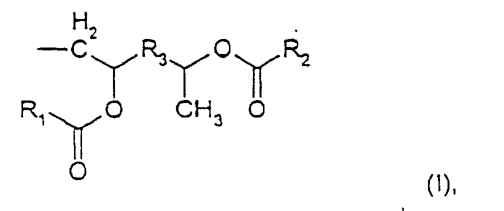 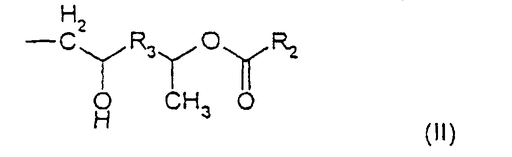 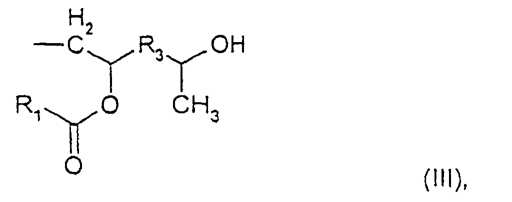 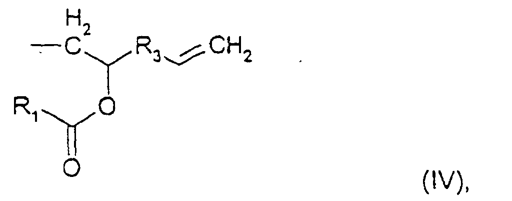 and the isomers thereof with the formulae: 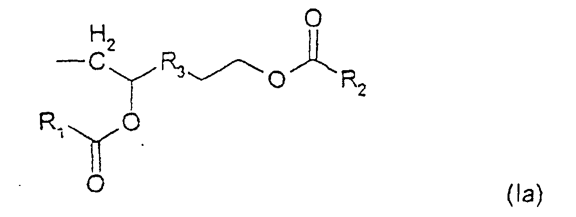 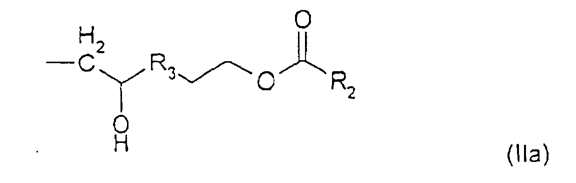 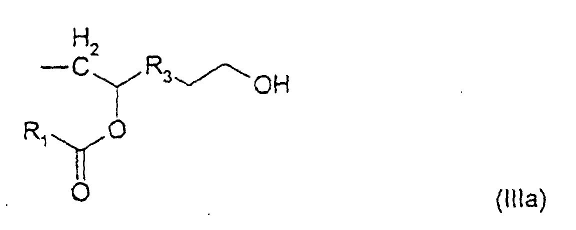 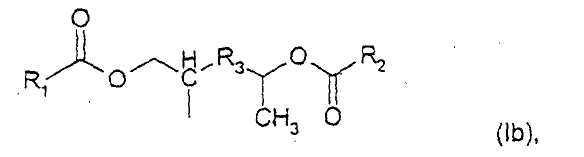 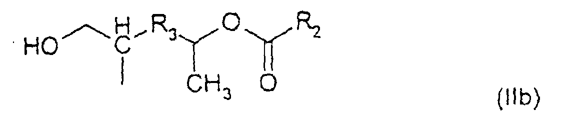 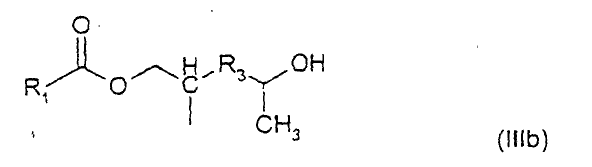 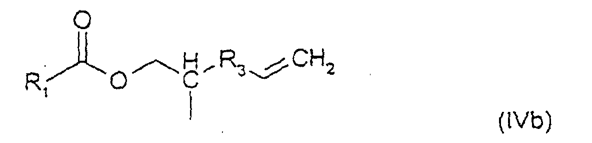 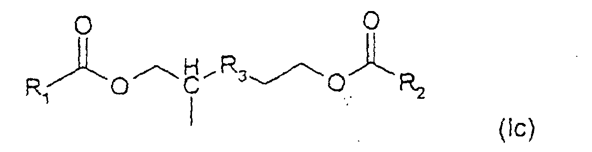 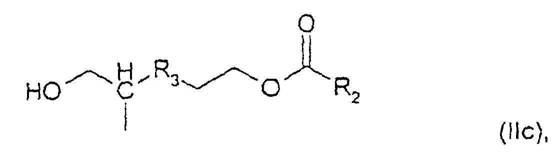 and 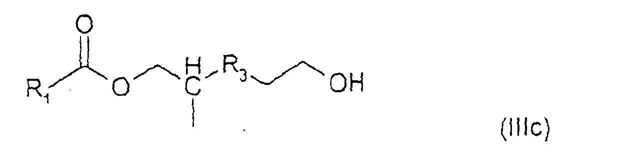 wherein,
R₁ and R₂ are independently selected from linear or branched, saturated or unsaturated C₆₋₂₂ hydrocarbyl,
R₃ is nothing or C₁₋₂₀ hydrocarbyl,
R₄ is C₁₋₆ alkyl, C₁₋₆ alkylene, or independent Z,
R₅ is H, C₁₋₆ alkyl, independent Z, or the residue of the quaternizing agent, such as C₁₋₃₀ alkyl or alkenyl, preferably, C₁₋₇ alkyl or alkenyl,
R₆ is C₁₋₆ alkyl or independent Z,
n is 1, 2 or 3 and
X⁻ is an ion selected from Cl⁻, Br, I⁻, F-, CH₃SO₄⁻, C₂H₅SO₄⁻, H₂PO₄⁻, HPO₄²⁻, PO₄³⁻, H₂PO₃⁻, HPO₃²⁻, H₂PO₂⁻, HPO₂²⁻, nitrate-, formate⁻, acetate⁻, propionate⁻, tartrate⁻ and benzoate⁻, wherein the total charge of the anions equals the total charge of the cations.

2. Compounds according to claim 1 of the formula 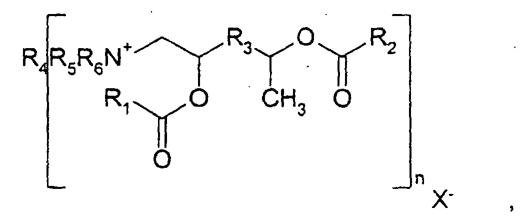 or isomers thereof,
wherein R₁-R₆, n, and X⁻ have the meaning given in claim 1.

3. Compounds according to claim 2 of the formula 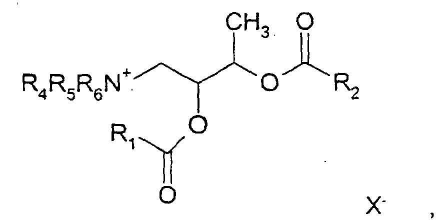 or isomers thereof,
wherein R₁, R₂, R₄-R₆ and X⁻ have the meaning given in claim 1.

4. Compounds according to any one of claims 1-3 wherein R₁ and R₂ are independently selected from linear or branched, saturated or unsaturated C₁₂₋₁₈ alkyl groups.

5. Compounds according to any one of the preceding claims, **characterized**
**in that** R₄ and R₆ are methyl.

6. Compounds according to any one of the preceding claims wherein X⁻ is chloride, methyl sulfate or ethyl sulfate.

7. Intermediates for making one or more of the compounds of the preceding claims according to the formula R₄[R₆NZ]ₙ, wherein R₄, R₆, n, and Z have the meaning given in claim 1.

8. Compositions comprising one or more of the compounds according to any one of the preceding claims.

9. A process to make the compounds of claim 1 comprising the steps of:
- reacting an unsaturated epoxide of the formula 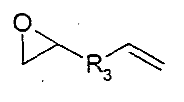 with an amine or protonated amine of the formula R₄[R₅R₆N]ₙ or R₄[R₅R₆N⁺H]ₙ X⁻, wherein R₃, R₄, R₅, R₆, n, and X⁻ have the meaning given in claim 1, and
- esterification of the intermediate with, on average, 1-2 moles of fatty acid derivatives, comprising the moieties R₁-C(O)-, R₂-C(O)- or mixtures thereof, per mole of OH groups of the intermediate,
- an optional conventional quaternization either before or after said esterification step.

10. A process according to claim 9, **characterized in that** a trialkylamine is reacted with the unsaturated epoxide.

11. A process according to claim 9 or 10, **characterized in that** a product according to any one of claims 2-6 is formed.

12. Use of a compound or composition according to any one of claims 1-8 as a fabric softener.

13. Use according to claim 12 wherein the compound is used in combination with a conventional performance booster selected from the group consisting of cationic and non-ionic surfactants.

## Patentansprüche

1. Quartäre Ammoniumverbindungen der Formel R₄[R₅R₆N⁺Z]ₙX⁻, wobei Z kovalent an das Stickstoffatom gebunden ist und ausgewählt ist aus der Gruppe der folgenden Formeln (I-IV): 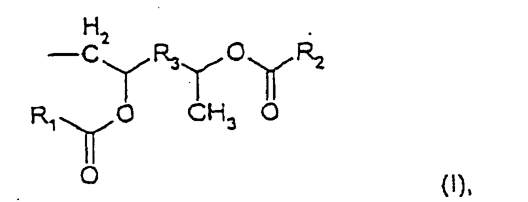 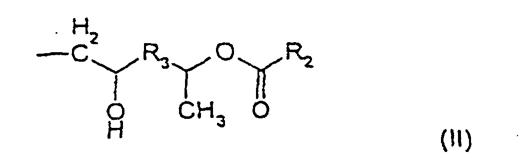 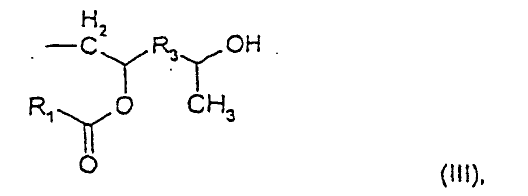 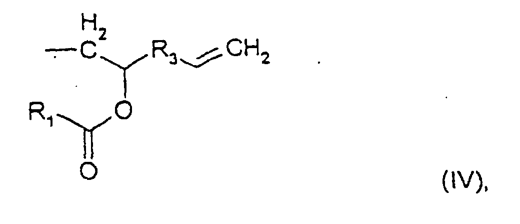 und deren Isomeren mit den Formeln: 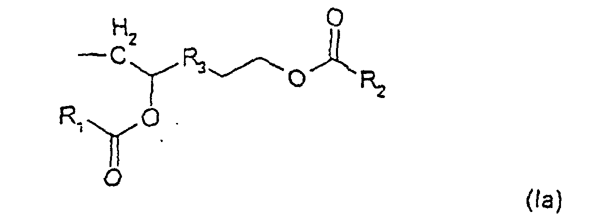 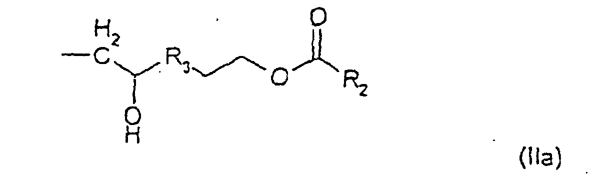 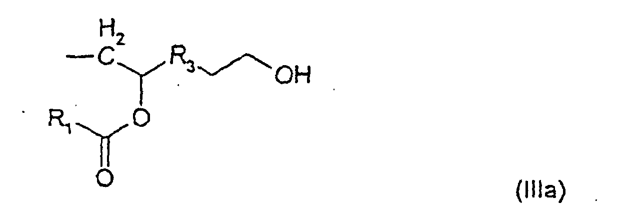 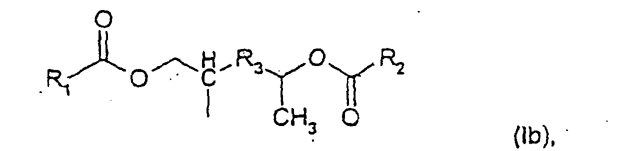 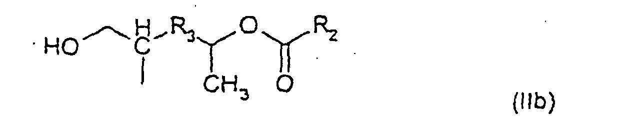 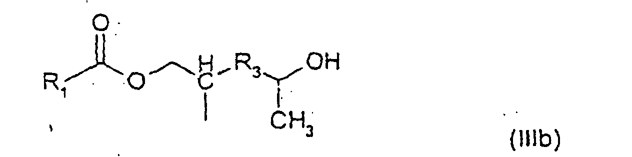 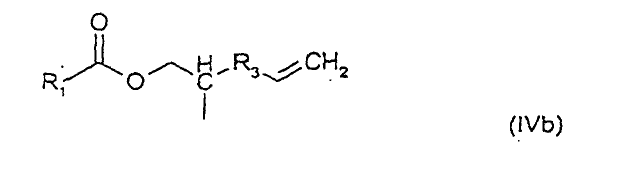 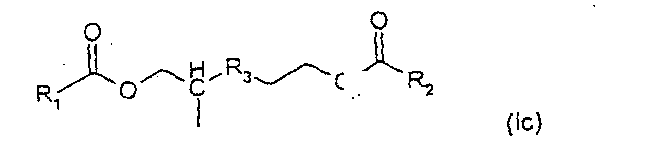 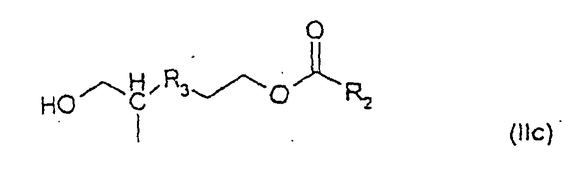 und 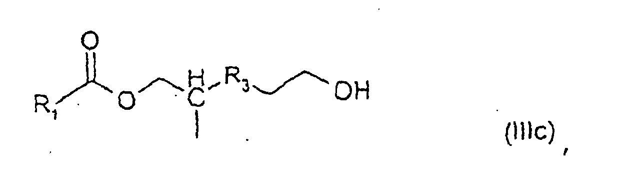 wobei
R₁ und R₂ unabhängig aus linearem oder verzweigtem, gesättigtem oder ungesättigtem C₆₋₂₂-Hydrocarbyl ausgewählt sind;
R₃ nichts oder C₁₋₂₀-Hydrocarbyl ist;
R₄ C₁₋₆-Alkyl, C₁₋₆-Alkylen oder unabhängiges Z ist;
R₅ H, C₁₋₆-Alkyl, unabhängiges Z oder der Rest des Quaternisierungsmittels, wie C₁₋₃₀-Alkyl oder -Alkenyl, vorzugsweise C₁₋₇-Alkyl oder -Alkenyl, ist;
R₆ C₁₋₆-Alkyl oder unabhängiges Z ist;
n = 1, 2 oder 3 ist; und
X⁻ ein Ion ist, das aus Cl⁻, Br⁻, I⁻, F⁻, CH₃SO₄⁻, C₂H₅SO₄⁻, H₂PO₄⁻, HPO₄²⁻, PO₄³⁻, H₂PO₃⁻, HPO₃²⁻, H₂PO₂⁻, HPO₂²⁻, Nitrat, Formiat, Acetat, Propionat, Tartrat und Benzoat ausgewählt ist, wobei die Gesamtladung der Anionen gleich der Gesamtladung der Kationen ist.

2. Verbindungen gemäß Anspruch 1 mit der Formel 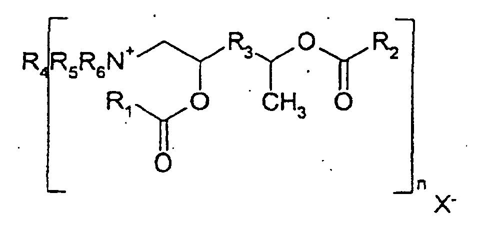 oder Isomere davon, wobei R₁-R₆, n und X⁻ die in Anspruch 1 angegebene Bedeutung haben.

3. Verbindungen gemäß Anspruch 2 mit der Formel 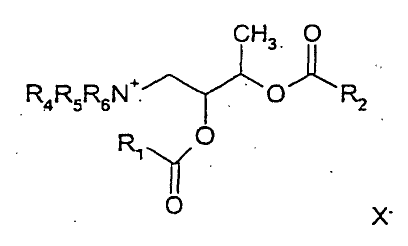 oder Isomere davon, wobei R₁, R₂, R₄-R₆ und X⁻ die in Anspruch 1 angegebene Bedeutung haben.

4. Verbindungen gemäß einem der Ansprüche 1-3, wobei R₁ und R₂ unabhängig aus linearen oder verzweigten, gesättigten oder ungesättigten C₁₂₋₁₈-Alkylgruppen ausgewählt sind.

5. Verbindungen gemäß einem der vorstehenden Ansprüche, **dadurch gekennzeichnet, dass** R₄ und R₆ Methyl sind.

6. Verbindungen gemäß einem der vorstehenden Ansprüche, wobei X-Chlorid, Methylsulfat oder Ethylsulfat ist.

7. Zwischenstufen für die Herstellung von einer oder mehreren der Verbindungen der vorstehenden Ansprüche gemäß der Formel R₄[R₆NZ]ₙ, wobei R₄, R₆, n und Z die in Anspruch 1 angegebene Bedeutung haben.

8. Zusammensetzungen, die eine oder mehrere der Verbindungen gemäß einem der vorstehenden Ansprüche umfassen.

9. Verfahren zur Herstellung der Verbindungen gemäß Anspruch 1, das die folgenden Schritte umfasst:
- Umsetzen eines ungesättigten Epoxids der Formel 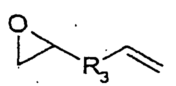 mit einem Amin oder protonierten Amin der Formel R₄[R₅R₆N]ₙ oder R₄[R₅R₆N⁺H]ₙX⁻, wobei R₃, R₄, R₅, R₆, n und X⁻ die in Anspruch 1 angegebene Bedeutung haben; und
- Veresterung der Zwischenstufe mit im Durchschnitt 1-2 mol Fettsäurederivaten, die die Struktureinheiten R₁-C(O)-, R₂-C(O)- oder Kombinationen davon umfassen, pro Mol OH-Gruppen der Zwischenstufe;
- gegebenenfalls eine herkömmliche Quaternisierung entweder vor oder nach dem Veresterungsschritt.

10. Verfahren gemäß Anspruch 9, **dadurch gekennzeichnet, dass** ein Trialkylamin mit dem ungesättigten Epoxid umgesetzt wird.

11. Verfahren gemäß Anspruch 9 oder 10, **dadurch gekennzeichnet, dass** ein Produkt gemäß einem der Ansprüche 2-6 gebildet wird.

12. Verwendung einer Verbindung oder Zusammensetzung gemäß einem der Ansprüche 1-8 als Weichmacher für einen Textilstoff.

13. Verwendung gemäß Anspruch 12, wobei die Verbindung in Kombination mit einem herkömmlichen Leistungsverstärker, der aus der Gruppe ausgewählt wird, die aus kationischen und nichtionischen Tensiden besteht, verwendet wird.

## Revendications

1. Composés d'ammonium quaternaire de formule R₄[R₅R₆N⁺Z]ₙ X⁻, dans laquelle Z est lié de manière covalente à l'atome d'azote et choisi parmi le groupe des formules suivantes (I-IV) 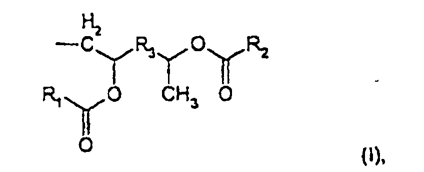 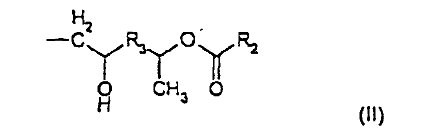 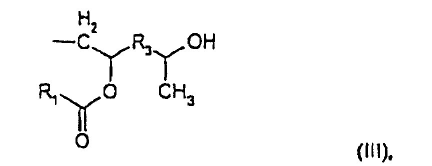 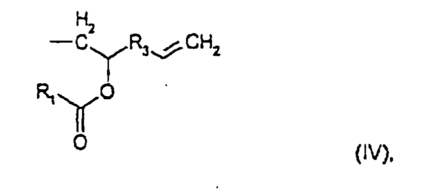 et les isomères de ceux-ci avec les formules : 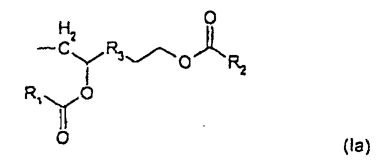 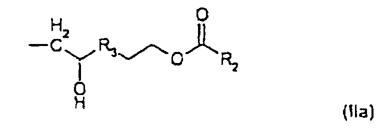 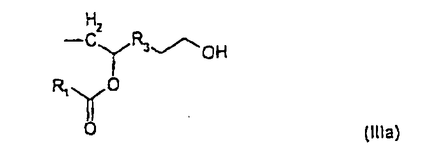 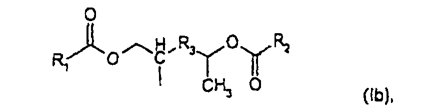 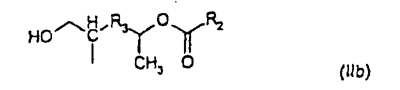 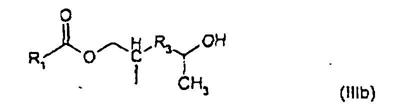 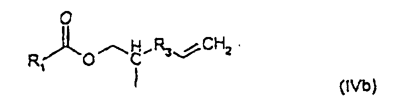 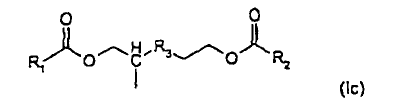 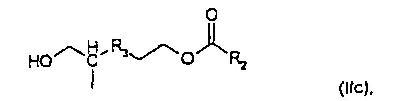 et 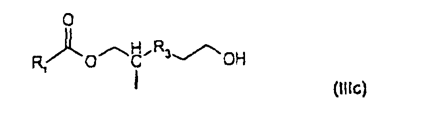 dans lesquelles
R₁ et R₂ sont indépendamment choisis parmi les hydrocarbyles en C₆₋₂₂ linéaires ou ramifiés, saturés ou insaturés,
R₃ est rien ou un hydrocarbyle en C₁₋₂₀,
R₄ est alkyle en C₁₋₆, alkylène C₁₋₆ ou Z indépendant,
R₅ est H, un alkyle en C₁₋₆, Z indépendant, ou le résidu de l'agent quaternisant, comme un alkyle ou alcényle en C₁₋₃₀, de préférence un alkyle ou alcényle en C₁₋₇,
R₆ est un alkyle en C₁₋₆ ou Z indépendant,
n est égal à 1, 2 ou 3, et
X⁻ est un ion choisi parmi le Cl⁻, Br⁻, I⁻, F⁻, CH₃SO₄⁻, C₂H₅SO₄⁻, H₂PO₄⁻, HPO₄²⁻, PO₄³⁻, H₂PO₃⁻, HPO₃²⁻, H₂PO₂⁻, HPO₂²⁻, nitrate⁻, formate⁻, acétate⁻, propionate⁻, tartrate⁻ et benzoate⁻, dans lesquels la charge totale des anions est égale à la charge totale des cations.

2. Composés selon la revendication 1, de formule 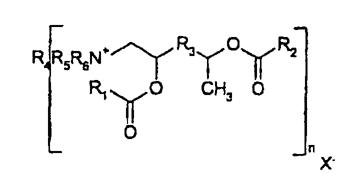 ou isomères de ceux-ci,
dans laquelle R₁ à R₆, n et X⁻ ont les significations données dans la revendication 1.

3. Composés selon la revendication 2, de formule 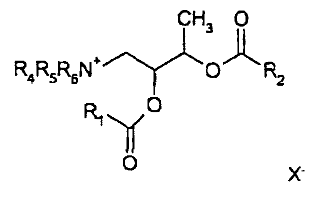 ou isomères de ceux-ci,
dans laquelle R₁, R₂, R₄ à R₆, et X⁻ ont les significations données dans la revendication 1.

4. Composés selon l'une quelconque des revendications 1 à 3, dans laquelle R₁ et R₂ sont choisis indépendamment parmi les groupes alkyle en C₁₂₋₁₈ linéaires ou ramifiés, saturés ou insaturés.

5. Composés selon l'une quelconque des revendications précédentes, **caractérisés en ce que** R₄ et R₆ sont un groupe méthyle.

6. Composés selon l'une quelconque des revendications précédentes, dans laquelle X⁻ est le chlorure, le sulfate de méthyle ou le sulfate d'éthyle.

7. Intermédiaires pour la préparation d'un ou de plusieurs composés des revendications précédentes, selon la formule R₄[R₆NZ]ₙ, dans laquelle R₄, R₆, n et Z ont les significations données dans la revendication 1.

8. Compositions comprenant l'un ou plusieurs des composés selon l'une quelconque des revendications précédentes.

9. Procédé de préparation des composés selon la revendication 1 comprenant les étapes consistant à :
- faire réagir un époxyde insaturé de formule 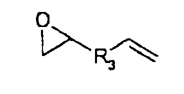 avec une amine ou une amine protonée de formule R₄[R₅R₆N]ₙ ou R₄[R₅R₆N⁺H]ₙ X⁻, dans laquelle R₃, R₄, R₅, R₆, n et X⁻ ont les significations données dans la revendication 1, et
- estérifier l'intermédiaire avec, en moyenne, 1 à 2 moles de dérivés d'acide gras, comprenant les groupes R₁-C(O)-, R₂-C(O)- ou des mélanges de ceux-ci, par mole de groupes OH de l'intermédiaire,
- éventuellement le quaterniser de manière usuelle soit avant soit après ladite étape d'estérification.

10. Procédé selon la revendication 9, **caractérisé en ce qu'**une trialkylamine réagit avec l'époxyde insaturée.

11. Procédé selon la revendication 9 ou 10, **caractérisé en ce que** le produit selon l'une quelconque des revendications 2 à 6 est formé.

12. Utilisation d'un composé ou d'une composition selon l'une quelconque des revendications 1 à 8 à titre d'adoucissant textile.

13. Utilisation selon la revendication 12, dans laquelle le composé est utilisé en combinaison avec un stimulateur de performance usuel choisi parmi le groupe constitué de tensioactifs cationiques et non ioniques.
